# EUROPEAN PATENT SPECIFICATION

(11) **EP 3 691 534 B1**
(45) Date of publication and mention of the grant of the patent: **29.11.2023**
(21) Application number: 18804682.5
(22) Date of filing: 05.10.2018
(51) Int. Cl.: A61B 8/08, A61B 8/00, A61N 7/02, A61B 6/03, A61B 6/12, A61B 6/00, A61B 5/055, G06T 3/00, G06T 7/20, A61B 5/00, G06T 7/30, G06T 7/32, G06T 7/70, G01R 33/48, G01R 33/56, A61B 90/00

(54) **ULTRASOUND THERAPY**
ULTRASCHALLTHERAPIE
THÉRAPIE PAR ULTRASONS

(30) Priority: 05.10.2017 US 201762568439 P
(43) Date of publication of application: 12.08.2020
(73) Proprietor: Insightec Ltd., 39120 Tirat-Carmel (IL)
(72) Inventor: ZADICARIO, Eyal, Tel Aviv-Yafo (IL); LEVY, Yoav, Hinanit, OT (IL); VORTMAN, Kobi, 34467 Haifa (IL)
(74) Representative: Dennemeyer & Associates S.A.
(86) International application number: PCT/IB2018/001246
(87) International publication number: WO 2019/069135

(56) References cited:
- US-A1- 2014 046 172

## Description

### TECHNICAL FIELD

The present invention relates, in general, to tracking moving tissue or organs, and, in particular, to tracking motion thereof during an ultrasound treatment planning and/or procedure.

### BACKGROUND

Tissue, such as a benign or malignant tumor or blood clot within a patient's skull or other body region, may be treated invasively by surgically removing the tissue or non-invasively by using, for example, thermal ablation. Both approaches may effectively treat certain localized conditions within the body, but involve delicate procedures to avoid destroying or damaging otherwise healthy tissue. Unless the healthy tissue can be spared or its destruction is unlikely to adversely affect physiological function, surgery may not be appropriate for conditions in which diseased tissue is integrated within healthy tissue.

Ultrasound therapy, as may be accomplished using focused ultrasound, has particular appeal for treating diseased tissue surrounded by or neighboring healthy tissue or organs because the effects of ultrasound energy can be confined to a well-defined target region. Ultrasonic energy may be focused to a zone having a cross-section of only a few millimeters due to relatively short wavelengths (e.g., as small as 1.5 millimeters (mm) in cross-section at one Megahertz (1 MHz)). Moreover, because acoustic energy generally penetrates well through soft tissues, intervening anatomy often does not pose an obstacle to defining a desired focal zone. Thus, ultrasonic energy may be focused at a small target in order to ablate diseased tissue without significantly damaging surrounding healthy tissue.

In addition, ultrasound may be utilized to open the blood-brain barrier (BBB) in the treatment of neurological diseases. The BBB, formed by layers of cells in the central nervous system (CNS), prevents large molecules from entering the brain parenchyma, and thus presents one of the largest obstacles to treating many brain diseases. Specifically, the BBB prevents many therapeutic agents, such as drugs and gene-therapy vectors, from reaching a patient's brain tissue. For example, treatments for CNS infections, neurodegenerative diseases, congenital enzyme defects and brain cancer are all hampered by the ability of the BBB to block passage of, inter alia, antibiotics, anti-retroviral drugs, enzyme replacement therapy, gene preparations and anti-neoplastic drugs. It is thus desirable to using ultrasound energy to temporarily and locally "open" the BBB to permit therapeutic quantities of these agents to access the affected brain tissue.

An ultrasound focusing system generally utilizes an acoustic transducer surface, or an array of transducer surfaces, to generate an ultrasound beam. The transducer may be geometrically shaped and positioned to focus the ultrasonic energy at a "focal zone" corresponding to the target tissue mass within the patient. During wave propagation through the tissue, a portion of the ultrasound energy is absorbed, leading to increased temperature and, eventually, to cellular necrosis - preferably at the target tissue mass in the focal zone. The individual surfaces, or "elements," of the transducer array are typically individually controllable, i.e., their phases and/or amplitudes can be set independently of one another (e.g., using a "beamformer" with suitable delay or phase shift in the case of continuous waves and amplifier circuitry for the elements), allowing the beam to be steered in a desired direction and focused at a desired distance, and the focal zone properties to be shaped as needed. Thus, the focal zone can be rapidly displaced and/or reshaped by independently adjusting the amplitudes and/or phases of the electrical signal input into the transducer elements.

The human body, however, is flexible and can move even when a patient is positioned to keep still (due to respiration, for example, or small involuntary movements); this can pose a considerable challenge to therapeutic efficacy and safety. One conventional approach implements a stereotactic frame to immobilize the head and fix it mechanically relative to the ultrasound transducer. This approach disadvantageously requires local anesthesia and pins that penetrate the skin and soft tissue in order to provide a solid interface with the skull. In addition, while suitable for a single treatment session, this approach is impractical for recurring therapy that involves multiple treatment sessions. Another approach utilizes a thermoplastic mask covering the patient's face that can secure the patient's head to a table in order to immobilize it. Although the thermoplastic mask obviates the need for local anesthesia and penetrating pins, it does not provide sufficiently rigid immobilization. As a result, significant treatment inefficiencies and damage to healthy tissue may occur. Moreover, covering the patient's face with a mask may cause varying levels of stress and anxiety. US2014/046172A1 discloses a method and apparatus for tracking a tumor position, which changes by the movement of a body.

Accordingly, there is a need for approaches that facilitate tracking the ultrasound target, and compensating for its motion, in real time during the treatment without the need to immobilize the patient's head against a stationary object.

### SUMMARY

Various embodiments of the present invention provide systems and methods for tracking the motion of a target or other object(s) of interest in real time during an ultrasound treatment planning and/or procedure, and based thereon correcting measurement information (e.g., the temperature and/or location) of the target and/or adjusting treatment parameter values to compensate for the target motion. In general, the technique utilizes a closed-loop feedback system providing information (e.g., a location and/or a temperature) about the target to an operator or to an automatic control system so as to allow for measurement correction and/or treatment adjustment/optimization. In various embodiments, the closed-loop feedback system involves tracking based on image content and utilizes two imaging modalities - a more accurate (e.g., higher resolution) but slower-rate modality (such as magnetic resonance imaging (MRI)) and a less accurate (e.g., lower resolution) but faster-rate modality (such as a camera or ultrasound imaging). For example, prior to and/or during the treatment, one or more MR images may be acquired to accurately identify the location of the target and/or generate a thermal map of the target at a given time. However, due to the complexity of image capture and processing, the MRI generally has a relatively low frequency and high latency (e.g., 10 image frames per second); because the patient may move during the image-acquisition and/or image-processing interval, it may be desirable to employ a second imaging system having a higher frequency and shorter latency to assist in identifying and locating the target. For example, ultrasound imaging (which may be activated every 10 ms to capture images) may involve obtaining images by application of low-energy ultrasound pulses (i.e., pulsed ultrasound that does little or no damage) to the target and analysis of the reflections therefrom. Image information provided by the ultrasound system may then be combined with the MR image information so as to determine, and account for, the target movement; this enables the target location to be accurately identified for creating a treatment plan as well as allowing real-time adjustment of the treatment plan during execution thereof, thereby improving treatment efficiency and safety.

To generate a thermal map of the target and/or its surrounding tissue rather than a conventional image, MR thermometry (or MR thermal imaging) may be employed. MR thermal imaging allows the target and/or its surrounding tissue to be continuously monitored during treatment, thereby avoiding latency, and facilitates correction for local differences in heat conduction and energy absorption so as to increase treatment efficiency and avoid damage to tissue surrounding the target region. Typically, in the context of MR thermometry, a baseline proton resonance frequency (PRF) phase image is acquired (typically before the treatment starts), and a second phase image is acquired during treatment, i.e., after the temperature has changed; the phase differences between the image acquired during treatment and the baseline image can be used to determine the change in temperature. In one embodiment, the images acquired using the faster-rate modality (e.g., the camera or ultrasound images) are utilized to adjust the baseline phase image and/or images acquired during treatment so as to eliminate (or at least reduce) artifacts resulting from target movement rather than temperature changes; this enables the MRI system to accurately measure the temperature at the target and/or non-target region in real time.

Thus, in various embodiments, the invention may be employed to detect movement of the target tissue in order to create an accurate treatment plan and facilitate efficient and safe treatment. In addition, the fast-rate modality may provide image information that is combined with a thermal map in order to simultaneously show image and temperature information in a single display that is updated in real-time. Further, the detected movement may be utilized to eliminate spurious phase differences that would otherwise distort the temperature.

In some embodiments, the MRI and/or ultrasound imaging modalities acquire images of one or more non-target objects that have specific (e.g., fixed) relative positions with respect to the target. By identifying the locations of the non-target objects, the target location can be inferred therefrom. Because non-target objects are utilized to infer the target location, the images thereof may be acquired at a faster rate with lower resolution to expedite image acquisition and processing, allowing the target location to be tracked in real time. In one embodiment, images of non-target objects are acquired using a camera, ultrasound imaging, or MRI with a faster scanning rate for each frame (e.g., by skipping some scan lines). Again, if target motion is identified, information characterizing the motion may be utilized to correct the temperature measurements and/or adjust treatment parameters to compensate. In addition, if the non-target objects are in sufficient proximity to the target, the MR imaging data of the non-target objects may be utilized to estimate the target temperature using MR thermometry. The non-target objects may be MRI fiducials, for example, including anatomical features (e.g., the patient's nose tip, ears, eye edges or upper jaw teeth) and/or MR trackers that are attached externally to the skull or, preferably, implanted sub-dermally and left in place during the entire duration of the treatment. In one embodiment, the implanted markers are small, flat pellets made of bio-compatible magnetic nanoparticles encapsulated in a thin bio-compatible plastic envelope.

In some embodiments, the ultrasound treatment involves microbubbles in order to, for example, facilitate BBB opening, enhance treatment effects or perform autofocusing. An acoustic-signal detector is implemented to measure acoustic signals from the microbubbles at the target and/or non-target region during treatment, and based thereon, a microbubble dose map (indicating the amount of microbubbles at various locations) or a temporal cavitation activity map (indicating the type(s), dose(s) and/or location(s) associated with the microbubble cavitation at various locations over time) may be generated. In some embodiments, the microbubble dose map or temporal cavitation activity map may be combined with the MR imaging data to generate an image depicting the spatial relationship between the target/non-target regions and the microbubbles or cavitation activities. Based on the combined image, the ultrasound parameter values (e.g., frequencies, amplitudes and/or phases) and/or microbubble characteristics (e.g., the agent type, size, concentration, ultrasound energy level for creating the microbubbles, location of the microbubble injection site, and/or the administering profile, such as the administering dose or timing) may be adjusted in order to achieve the treatment goal.

Accordingly, in one aspect, the invention pertains to a system for treating an anatomical target according to claim 1.

In one implementation, the controller is further configured to activate the treatment apparatus based at least in part on the treatment plan.

The first or second imaging modality may be further configured acquire the second series of images of the second imageable object, and the controller may be further configured to track movement of the anatomical target based at least in part on the second series of images of the second imageable object. Alternatively, the system may further include the third imaging modality for acquiring the second series of images of the second imageable object and the controller is further configured to track movement of the anatomical target based at least in part on the second series of images of the second imageable object. In some embodiments, the controller is further configured to track movement of the anatomical target based at least in part on a predicted movement of the anatomical target and/or the second imageable object.

The first imageable object may include the anatomical target. Alternatively, the first imageable object is different from the anatomical target, and the controller is further configured to determine the location of the first imageable object in each of the series of images acquired in step (c), and infer a location of the anatomical target based on the determined location of the first imageable object.

The controller is configured to update the information associated with the anatomical target based on the tracked movement thereof. The image(s) of the anatomical target include(s) one or more baseline phase images, and the controller is further configured to subtract a phase shift associated with the tracked movement from the baseline phase image(s) so as to generate a thermal map of the anatomical target. In one embodiment, the first imaging modality further acquires a reference library including a series of reference images of the first imageable object. The controller is further configured to (i) compare one of the images of the first imageable object acquired using the second imaging modality against the reference images in the reference library to identify a best-matching reference image and (ii) determine the phase shift based at least in part on the identified best-matching reference image.

The second imaging modality may include an MRI apparatus; the MRI apparatus is configured to acquire the image(s) of the anatomical target at a relatively faster scanning rate and to acquire the series of images of the first imageable object at a relatively slower scanning rate. Alternatively, the second imaging modality may include a camera. In some embodiments, the second imaging modality includes an ultrasound apparatus. In addition, the treatment apparatus may include an ultrasound apparatus that is the same or different from the second imaging modality. In one embodiment, the ultrasound apparatus includes multiple transducer elements, and the treatment plan specifies an amplitude, a frequency, a phase, a direction and/or an activation time associated with one or more of the transducer elements.

As used herein, the term "substantially" means ±10%, and in some embodiments, ±5% of the peak intensity. Reference throughout this specification to "one example," "an example," "one embodiment," or "an embodiment" means that a particular feature, structure, or characteristic described in connection with the example is included in at least one example of the present technology. Thus, the occurrences of the phrases "in one example," "in an example," "one embodiment," or "an embodiment" in various places throughout this specification are not necessarily all referring to the same example. Furthermore, the particular features, structures, routines, steps, or characteristics may be combined in any suitable manner in one or more examples of the technology. The headings provided herein are for convenience only and are not intended to limit or interpret the scope or meaning of the claimed technology.

### BRIEF DESCRIPTION OF THE DRAWINGS

In the drawings, like reference characters generally refer to the same parts throughout the different views. Also, the drawings are not necessarily to scale, with an emphasis instead generally being placed upon illustrating the principles of the invention. In the following description, various embodiments of the present invention are described with reference to the following drawings, in which:
FIG. 1 illustrates a focused ultrasound system in accordance with various embodiments;
FIG. 2 illustrates an exemplary MRI apparatus in accordance with various embodiments of the present invention;
FIGS. 3A and 3B are flow charts illustrating approaches for tracking the target location and creating a treatment plan based thereon in accordance with various embodiments of the present invention;
FIG. 3C is a flow chart illustrating an approach for tracking the target location and adjusting the treatment plan based thereon in accordance with various embodiments of the present invention; and
FIGS. 4A and 4B depict approaches for determining whether the target has undergone significant movement in accordance with various embodiments of the present invention.

### DETAILED DESCRIPTION

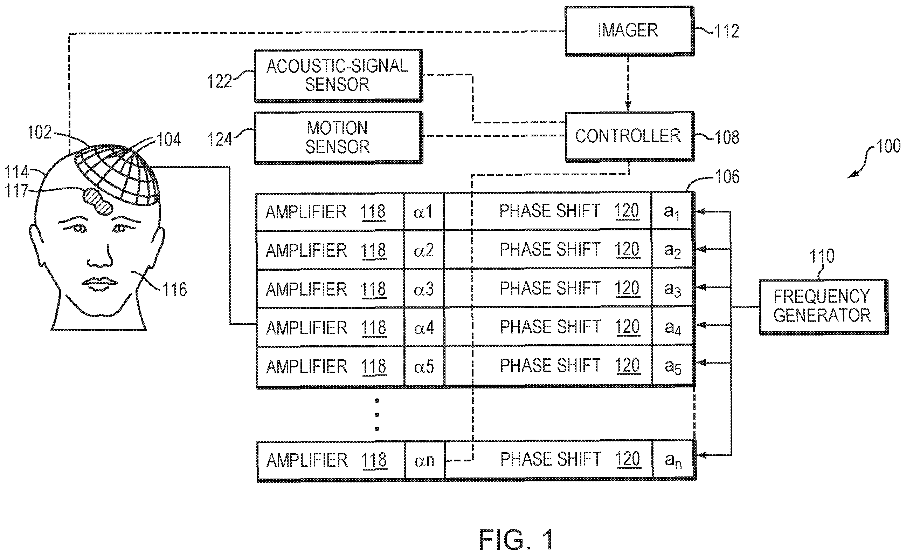

FIG. 1 illustrates an exemplary ultrasound therapy system 100 for focusing ultrasound within a patient's brain through the skull. One of ordinary skill in the art, however, will understand that the ultrasound system 100 described herein may be applied to any part of the human body. In various embodiments, the system 100 includes a phased array 102 of transducer elements 104, a beamformer 106 driving the phased array 102, a controller 108 in communication with the beamformer 106, and a frequency generator 110 providing an input electronic signal to the beamformer 106. In various embodiments, the system further includes an imager 112, such as an MRI device, a computer tomography (CT) device, a positron emission tomography (PET) device, a single-photon emission computed tomography (SPECT) device, or an ultrasonography device, for acquiring information (e.g., the geometry, material characteristics, anatomical characteristics, etc.) of the brain and/or skull 114 of a patient 116.

The array 102 may have a curved (e.g., spherical or parabolic) shape suitable for placement on the surface of the skull 114 or a body part other than the skull, or may include one or more planar or otherwise shaped sections. Its dimensions may vary, depending on the application, between millimeters and tens of centimeters. The transducer elements 104 of the array 102 may be piezoelectric ceramic elements or silicon-based elements, and may be mounted in any other material suitable for damping the mechanical coupling between the elements 104. Piezo-composite materials, or generally any materials (e.g., silicon devices) capable of converting electrical energy to acoustic energy, may also be used. To assure maximum power transfer to the transducer elements 104 and minimal reflections, the elements 104 may be configured for a specific (i.e., matching) electrical resonance (e.g., 50 Ω).

The transducer array 102 is coupled to the beamformer 106, which drives the individual transducer elements 104 so that they collectively produce a focused ultrasonic beam or field. For n transducer elements, the beamformer 106 may contain n driver circuits, each including or consisting of an amplifier 118 and a phase delay circuit 120; drive circuit drives one of the transducer elements 104. The beamformer 106 receives a radio frequency (RF) input signal, typically in the range from 0.1 MHz to 10 MHz, from the frequency generator 110, which may, for example, be a Model DS345 generator available from Stanford Research Systems. The input signal may be split into n channels for the n amplifiers 118 and delay circuits 120 of the beamformer 106. In some embodiments, the frequency generator 110 is integrated with the beamformer 106. The radio frequency generator 110 and the beamformer 106 are configured to drive the individual transducer elements 104 of the transducer array 102 at the same frequency, but at different phases and/or different amplitudes.

The amplification or attenuation factors α₁-αₙ and the phase shifts a₁-aₙ imposed by the beamformer 106 serve to transmit and focus ultrasonic energy through the patient's skull 114 onto a selected region (e.g., the target region 117) of the patient's brain, and account for wave distortions induced in the skull 114 and soft brain tissue. The amplification factors and phase shifts are computed using the controller 108, which may provide the computational functions through software, hardware, firmware, hardwiring, or any combination thereof. For example, the controller 108 may utilize a general-purpose or special-purpose digital data processor programmed with software in a conventional manner, and without undue experimentation, in order to determine the phase shifts and amplification factors necessary to obtain a desired focus or any other desired spatial field patterns. In certain embodiments, the computation is based on detailed information about the characteristics (e.g., structure, thickness, density, etc.) of the skull 114 and their effects on propagation of acoustic energy. Such information may be obtained from the imager 112 as further described below. Image acquisition may be three-dimensional or, alternatively, the imager 112 may provide a set of two-dimensional images suitable for reconstructing a three-dimensional image of the skull 114 from which thicknesses and densities can be inferred. Image-manipulation functionality may be implemented in the imager 112, in the controller 108, or in a separate device.

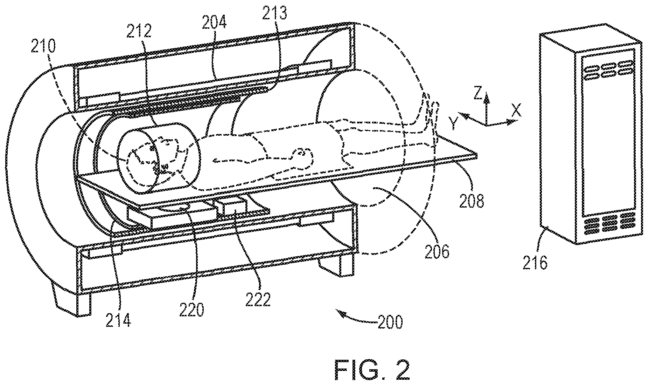

In various embodiments, the imager 112 is an MRI apparatus. With reference to FIG. 2, the MRI apparatus 200 may include a cylindrical electromagnet 204, which generates the requisite static magnetic field within a bore 206 of the electromagnet 204. During medical procedures, a patient is placed inside the bore 206 on a movable support cradle 208. A region of interest 210 (e.g., the patient's head) may be positioned within an imaging region 212 in which the electromagnet 204 generates a substantially homogeneous field. A set of cylindrical magnet field gradient coils 213 may also be provided within the bore 206 and surrounding the patient. The gradient coils 213 generate magnetic field gradients of predetermined magnitudes, at predetermined times, and in three mutually orthogonal directions. With the field gradients, different spatial locations can be associated with different precession frequencies, thereby giving an MR image its spatial resolution. An RF transmitter coil 214 surrounding the imaging region 212 emits RF pulses into the imaging region 212, and receives MR response signals emitted from the region of interest 210. (Alternatively, separate MR transmitter and receiver coils may be used.)

The MRI apparatus 200 generally includes an MRI controller 216 that controls the pulse sequence, i.e., the relative timing and strengths of the magnetic field gradients and the RF excitation pulses and response detection periods. The MRI controller 216 may be combined with the transducer controller 108 into an integrated system control facility.

The MR response signals are amplified, conditioned, and digitized into raw data using a conventional image-processing system, and further transformed into arrays of image data by conventional methods known to those of ordinary skill in the art. The image-processing system may be part of the MRI controller 216, or may be a separate device (e.g., a general-purpose computer containing image-processing software) in communication with the MRI controller 216 and/or the transducer controller 108. Because the response signal is tissue- and temperature-dependent, it can be processed to identify the treatment target region (e.g., a tumor to be destroyed by heat) 117 in the image, as well as to compute a temperature map from the image. Further, the acoustic field resulting from ultrasound application may be monitored in real time, using, e.g., thermal MRI or MR-based acoustic radiation force imaging. Thus, using MRI data, the ultrasound transducer 102 may be driven so as to focus ultrasound into (or near) the treatment region 117 while the temperature of the target and surrounding tissues and/or the acoustic field intensity are being monitored.

As described above, MR imaging can provide a non-invasive means of quantitatively monitoring *in vivo* temperatures. This is particularly useful in MR-guided thermal therapy (e.g., MR-guided focused ultrasound (MRgFUS) treatment), where the temperature of the target region 117 should be continuously monitored in order to assess the progress of treatment and correct for local differences in heat conduction and energy absorption to avoid damage to tissues surrounding the target. Monitoring (e.g., measurement and/or mapping) of the temperature is generally based on MR imaging (referred to as MR thermometry or MR thermal imaging) in conjunction with suitable image-processing software.

Among various methods available for MR thermometry, the PRF shift method is often the method of choice due to its linearity with respect to temperature change, near-independence from tissue type, and the high spatial and temporal resolution of temperature maps obtained therewith. The PRF shift method is based on the phenomenon that the MR resonance frequency of protons in water molecules changes linearly with temperature (with a constant of proportionality that, advantageously, is relatively constant among tissue types). Since the frequency change with temperature is small, only -0.01 ppm/°C for bulk water and approximately -0.0096 to -0.013 ppm/°C in tissue, the PRF shift is typically detected with a phase-sensitive imaging method in which the imaging is performed twice: first to acquire a baseline PRF phase image prior to a temperature change and then to acquire a second phase image after the temperature change - i.e., a treatment image - thereby capturing a small phase change that is proportional to the change in temperature. A map of temperature changes may then be computed from the (reconstructed, i.e., real-space) images by determining, on a pixel-by-pixel basis, phase differences between the baseline image and the treatment image, and converting the phase differences into temperature differences based on the PRF temperature dependence while taking into account imaging parameters such as the strength of the static magnetic field and echo time (TE) (e.g., of a gradient-recalled echo).

In various embodiments, prior to an ultrasound treatment procedure, the MRI apparatus 200 acquires one or more images of the target and/or non-target regions. The acquired MR images provide accurate locational information for the purpose of treatment planning, as well as baseline phase maps for determining the temperature at the target and/or non-target regions during treatment. In some embodiments, the ultrasound treatment involves microbubbles. For example, microbubbles may be generated by acoustic energy and/or introduced by systemic injection for BBB opening, enhancing treatment effects, and/or autofocusing. An acoustic-signal detector (e.g., the transducer array 102 and/or an acoustic-signal sensor 122) may be implemented to acquire information about the microbubbles at the target/non-target regions; the acquired microbubble information may then be analyzed to create a microbubble dose map or a temporal cavitation activity map. For example, during treatment, the acoustic response emanating from the microbubbles may be detected using the acoustic-signal detector. By analyzing the detected signals, the controller 108 may determine the amount of microbubbles and/or the type(s), dose(s) and/or location(s) associated with the microbubble cavitation. Based thereon, a microbubble dose map and/or a cavitation activity map over time may be created. Approaches to measuring acoustic response from the microbubbles and determining the microbubble amount and cavitation type(s), dose(s) and/or location(s) are provided, for example, in International Application No. PCT/IB2018/000774 (filed on May 22, 2018) and U.S. Patent Application No. 15/415,351 (filed on January 25, 2017).

In some embodiments, the measured acoustic signals from the microbubbles are combined with the MR images of the target/non-target region so as to generate a combined image illustrating the spatial relationship between the locations of the target/non-target regions and the locations of the microbubbles or cavitation activities. Based on the combined image, the ultrasound parameter values (e.g., frequencies, amplitudes and/or phases) and/or microbubble characteristics (e.g., an agent type, a size, a concentration, an ultrasound energy level for creating the microbubbles, a location of a microbubble injection site, and/or an administering profile, such as an administering dose or timing) may be adjusted for the purposes of BBB opening, enhancing ultrasound treatment and/or autofocusing. Because combination of the MR imaging data with the microbubble dose map or cavitation activity map may involve different imaging modalities (e.g., ultrasound imaging and MR imaging), it may be necessary to register their respective coordinate systems; exemplary registration approaches are provided, for example, in U.S. Patent No. 9,934,570.

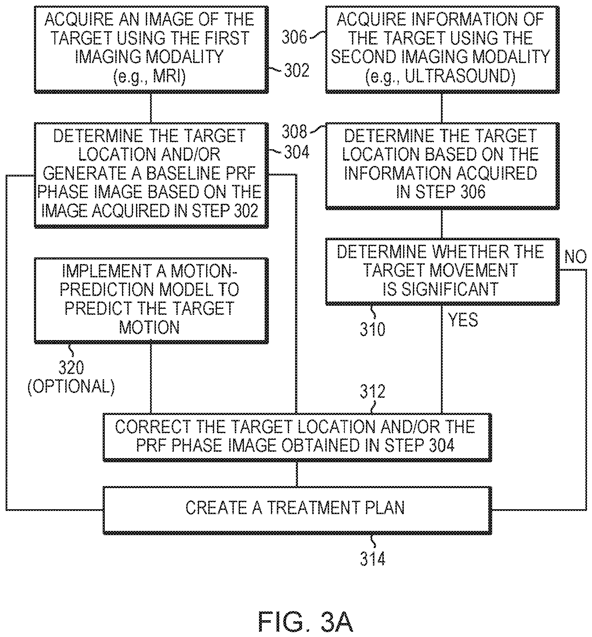

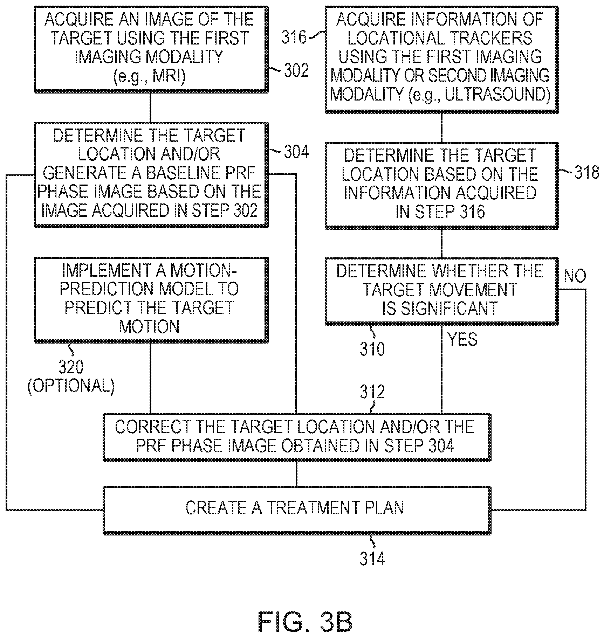

Acquisition of the MR imaging data, however, may take from a couple of minutes up to tens of minutes, and moreover, because the patient's motions (e.g., breathing or turning motions) during this period may significantly change the phase background and/or target/non-target locations, measurements based on the MR imaging data may be inaccurate. Accordingly, it may be necessary to track the target location and compensate for its motion in real time so as to improve treatment efficiency of the target tissue and avoid damage to the non-target tissue. FIGS. 3A and 3B illustrate various procedures for motion tracking of the target/non-target regions and, based thereon, correcting the measurement information and/or creating a treatment plan in accordance with various embodiments. For ease of reference, the following description only refers to target tracking; it should be understood, however, that the same approaches generally apply as well to the tracking of non-target regions, such as organs vulnerable to damage from the therapeutic beam, tissue surrounding the target, anatomical MRI fiducials (e.g., the patient's nose tip, ears, eye edges or upper jaw teeth having fixed positions with respect to the target) or other objects of interest (e.g., MR trackers that are externally attached to the skull 114 and/or sub-dermally implanted inside the skull 114). Accordingly, as used herein, the "non-target" objects may broadly include any anatomical regions outside the target region and/or any non-anatomical objects. In various embodiments, preparatory steps 302-316 are performed for creating the treatment plan prior to treatment. In a first preparatory step 302, one or more MR images of the target tissue are acquired as described above. In a second preparatory step 304, the acquired images are analyzed by the MRI controller 216 that implements conventional image-analysis software to determine the location of the target tissue and anatomical properties of the target/non-target tissue, and/or generate a baseline PRF phase image for MR thermometry. In various embodiments, during acquisition of the MR image(s), a second imaging system having a relatively faster frequency and shorter latency (compared with the MRI system) is activated to periodically acquire information (e.g., an image) of the target so as to track the movement thereof in real time (in a step 306). For example, the ultrasound transducer 102 may transmit sufficiently low-power ultrasound waves at 100 Hz to the target location (estimated in step 304) and receive the waves reflected therefrom. Preferably, the sufficiently low-power waves do not cause any significant clinical effects on the target. As used herein, "significant clinical effect" means having an undesired (and sometimes the lack of a desired) effect on tissue that is considered significant by clinicians, e.g., the onset of damage thereto or other clinically adverse effect, whether temporary or permanent. In various embodiments, the transducer 102 may possess both transmit and receive capabilities. For example, each transducer element 104 may alternate between transmitting and receiving ultrasound waves. Alternatively, some transducer elements 104 may transmit the ultrasound waves while other transducer elements 104 may receive the reflected waves at the same time. The transmitting and receiving regions of the transducer array may be configured in different suitable patterns and shapes. Approaches to configuring the transducer array 102 for transmitting acoustic signals to the target/non-target regions and measuring the acoustic signals reflected therefrom are provided, for example, in U.S. Patent Application No. 62/681,282, filed on June 6, 2018.

Additionally or alternatively, the acoustic signals from the target/non-target regions may be measured using the acoustic-signal sensor 122, which then transmits the measured signals to the controller 108 for analysis.

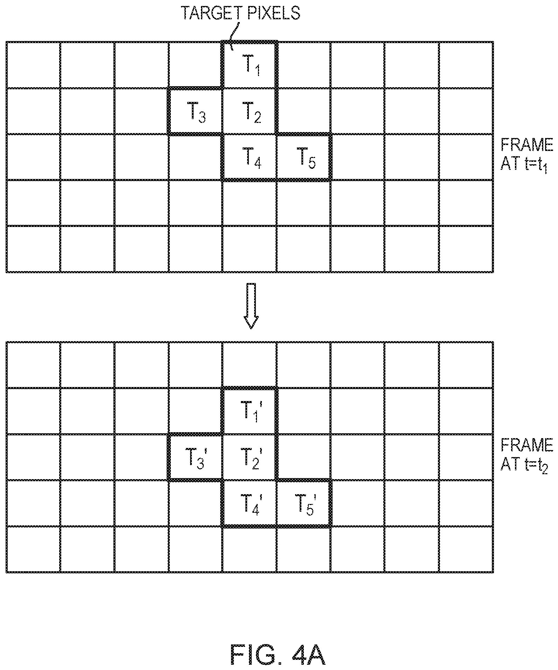

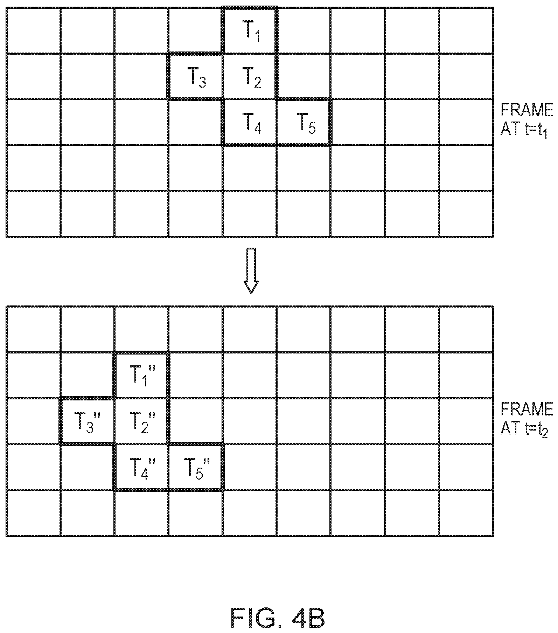

Based on the measured wave reflection signals, the controller 108 may create an image of the target and/or non-target regions; based thereon, the target location can be identified manually by an operator or automatically, using conventional techniques, by the controller 108 (in a step 308). For example, the ultrasound image may be processed by any of a number of feature-detection or tracking methods known to those of skill in the art to determine coordinates associated with the target. The target location may be determined in absolute coordinates within the image frame (e.g., in terms of row and column numbers) or generally in the ultrasound coordinate system using, e.g., edge detection or blob detection. The ultrasound imaging procedure may be periodically performed (e.g., every 100 ms) until acquisition of the MR image(s) is complete. In various embodiments, the target locations in two consecutive ultrasound images are compared on a pixel-by-pixel basis to determine the target movement (in a step 310). If the shift of target pixels therebetween falls below a predetermined threshold (in the aggregate or individually), it can be assumed that no target movement has occurred and no correction is required for the MR imaging data. For example, the threshold for individual target-pixel shift is two pixels, and the threshold for aggregated target-pixel shift is ten pixels. Referring to FIG. 4A, the target region may be first identified at target pixels T₁-T₅ in the first ultrasound image acquired at time ti; in the next ultrasound image acquired at time t₂, the target location is shifted to target pixels Ti'-T5'. Because each of the target pixels T₁-T₅ has shifted one pixel between the two images (smaller than the predetermined two-pixel-shift threshold) and the aggregated shift of the target pixels is five pixels (smaller than the predetermined ten-pixel-shift threshold), the target movement is deemed insignificant. In contrast, when the individual shift and/or aggregated shift of the target pixels exceeds the predetermined threshold(s), the target may be determined to have moved and a correction to the MR imaging data is necessary. For example, referring to FIG. 4B, if the target in the ultrasound image acquired at time t₂ is identified at target pixels T₁"-T₅", each target pixel is shifted three pixels (larger than the predetermined two-pixel-shift threshold) and the aggregated shift of the target region is fifteen pixels (larger than the predetermined ten-pixel-shift threshold). As a result, the target movement is considered sufficiently significant, and the MR imaging data may be corrected based thereon.

The thresholds may be determined based on the types, material properties, and/or locations of the target/non-target tissue. For example, if the non-target tissue next to the target region is a sensitive and/or important organ, the risk of damaging the non-target organ is high, and the need for treatment accuracy is heightened. Consequently, in this situation, the predetermined threshold corresponding to the maximal allowed pixel shift between target pixels in two consecutive ultrasound images (without the need for correcting the MR imaging data) may be smaller than for the situation where non-sensitive and/or clinically unimportant non-target tissue surrounds the target region. Thus, in one implementation, the thresholds are determined by the controller 108 based on, for example, the anatomical properties of the target/non-target tissue acquired in step 304 and/or a tissue model characterizing the material properties of the target and/or non-target tissue. Approaches to creating the tissue model are described, for example, in International Application No. PCT/IB2017/001689, filed on December 13, 2017.

In various embodiments, once the target movement is detected by ultrasound imaging, this information is employed to correct the MR imaging data (in a step 312). For example, the target location identified in the MR imaging data and/or the baseline PRF phase map provided by the MR imaging data may be adjusted to reflect the target movement. Again, the ultrasound imaging data may be registered with the MR imaging data using the approaches described above so as to convert the detected target movement from the ultrasound coordinates to MR coordinates. For example, image registration may be as simple as a one-to-one pixel mapping between the ultrasound and MR systems; thus, upon determining that the target location has shifted three pixels in the ultrasound coordinates (as shown in FIG. 4B), the controller may correct the target location in the MR imaging data acquired in step by 302 by shifting the target location therein by three pixels in the same directions (i.e., one pixel down and two pixels left).

In addition, a model or a look-up table that converts or maps target motions to phase shifts in the PRF phase image may be utilized to determine the phase shift corresponding to the detected target movement; this phase shift is then subtracted from the PRF baseline image created in step 304. In some embodiments, a reference library of phase images covering the anticipated range of target motion and providing baseline PRF phase images corresponding to the temperature in the target region is obtained prior to treatment. The detected target movement (converted into the coordinates of the MR system) is then compared against the baseline phase images in the reference library to identify the best match. The identified best-matching reference baseline image then serves as the corrected MR imaging data. Exemplary approaches to obtaining the reference library and identifying the best-matching reference image are described in U.S. Patent Publication No. 2015/0016682. Based on the adjusted/corrected MR imaging data, the controller 108 may create a treatment plan (in a step 314) for effectively treating the target. For example, the controller 108 may first apply the tissue model to obtain the material properties of the identified target and/or surrounding tissue (such as their heat sensitivities and/or tolerable thermal energies). The treatment plan may then specify a treatment goal including an ablation temperature at the target region and/or the maximum tolerable temperature at the non-target region. A computational physical model that simulates ultrasound field aberrations resulting from, for example, beams traversing inhomogeneous intervening tissue, transducer geometry and/or acoustic field design (e.g., for refocusing purposes) can be used to inversely compute the required parameter values (e.g., amplitudes, frequencies, phases, directions and/or activation time associated with the transducer elements, or time intervals between consecutive series of sonications) associated with the transducer elements based on the location and ablation temperature of the target region and/or the maximum tolerable temperature at the non-target region. These ultrasound parameter values may be included in the treatment plan as well. The treatment plan may be stored in memory accessible to the ultrasound controller 108 and/or the MRI controller 216 for retrieval during treatment. Exemplary approaches for creating the tissue model and physical model, simulating the ultrasound aberrations and treatment effects, and creating the treatment plan are described in U.S. Patent Publication No. 2015/0359603 and International Application Nos. PCT/IB2017/001689 (filed on December 13, 2017) and PCT/IB2018/000834 (filed on June 29, 2018).

Because the ultrasound reflections produce images at a high rate, the target may be monitored in real time (or substantially in real time). Therefore, the target is tracked using two separate measurement modalities - a more accurate but slower-rate modality (such as MR imaging) and a less accurate but faster-rate modality (such as ultrasound imaging). Each modality provides a distinct feedback loop (whose information may be separately utilized or combined) to determine the target information (e.g., a location or a temperature). It should be noted that while the examples herein describe an implementation using MRI and ultrasound systems, any two suitable measurement systems may be used in the approach described above, so long as they operate at different rates. For example, a camera may be employed to acquire less accurate but faster-rate images.

Alternatively, referring to FIG. 3B, the fast-loop and slow-loop tracking may be accomplished using the same imaging modality. For example, the MRI system 200 may acquire data associated with locational trackers (e.g., MR fiducials) that have specific (e.g., fixed) relative locations with respect to the target region and can be tracked by MR imaging (in a step 316); based thereon, the target location may be inferred (in a step 318). The MR fiducials may include or consist of anatomical features of the patient's body (e.g., the nose tip, ears, eye edges or upper jaw teeth). Additionally or alternatively, the MR fiducials may be MR trackers that are attached externally to the skull and/or implanted sub-dermally in the patient's head. In one embodiment, the implanted markers are small, flat pellets made of bio-compatible magnetic nanoparticles encapsulated in a thin bio-compatible plastic envelope. Because the MR fiducials serve merely to indicate the target location and no detailed image of them is necessary, the images of the MR fiducials may be obtained at a higher scanning rate with a lower resolution for reducing the image acquisition/processing time. Accordingly, during acquisition of the target images, the MRI system 200 may periodically (e.g., every 5 target-image frames) acquire images of the MR fiducials. Again, if the locations of the MR fiducials or the inferred target locations between two consecutive MR fiducial images differ by more than the predetermined threshold, the MR imaging data of the target acquired at the slow rate may be corrected as described above.

In one implementation, a reference library of the MR fiducial images and their associated phase images is established prior to treatment. During treatment, each acquired MR fiducial image may be compared against the MR fiducial images in the reference library to identify the best-matching image thereof; subsequently, the phase image associated with the best-matching MR fiducial image can be identified. Thus, by comparing the phase images associated with the identified best-matching MR fiducial images in two consecutive frames, the phase shift resulting from the MR fiducial movement can be computed, and the MR imaging data of the target region can be corrected. In some embodiments, the MR fiducials are in sufficient proximity to the target (e.g., within a few millimeters); the temperature at the MR fiducial locations may be approximately the same as the temperature at the target location. Therefore, the phase images of the MR fiducials may be utilized to estimate the target temperature using MR thermometry as described above.

Additionally, a motion-prediction model may be employed to predict the target motion prior to and/or during treatment. For example, natural movements of the patient's body (such as the regular heaving and ebbing of the abdominal region during a respiratory cycle) are predictable. Other potential movements of the patient's body (e.g., slight turning and/or tilting of the head or sliding of the hands or arms) may be anticipated. Accordingly, the motion-prediction model may include a predicted trajectory within a range of movement for the target region. The target region may follow and repeat the predicted trajectory in substantial synchronization with the patient's respiratory cycles. If it is anticipated that the patient might move his head, hands, or arms, the extent or range of such movements may also be estimated by the motion-prediction model. The predicted target motion and the measured target movement using the fast-rate imaging modality and/or the locational trackers may then be employed to correct the imaging data of the target acquired at the slow rate.

In various embodiments, the treatment plan specifies various ultrasound parameter values (e.g., amplitudes, frequencies, phases, directions and/or activation time associated with the transducer elements, or time intervals between consecutive series of sonications) so that a focal zone having a desired property (e.g., a location, a size, a shape and/or an acoustic intensity) is created at the target to produce the desired therapeutic effect. In addition, the treatment plan may include a minimal temperature to be achieved in the target region for ensuring effective treatment and/or a maximal allowable temperature in the non-target region for avoiding damage to the non-target tissue. In some embodiments, the treatment plan further specifies a risk level associated with the non-target region based on the type and/or location thereof. For example, if the non-target organ next to the target region is a sensitive and/or critical organ, the treatment plan may specify a smaller maximal movement of the target region that will result in suspension of treatment. If the treatment involves microbubble cavitation, the treatment plan may specify the ultrasound parameter values for creating the microbubbles and/or causing microbubble cavitation at the target, an agent type, a size, a concentration, a location of a microbubble injection site, and/or an administering profile (e.g., an administering dose or timing) associated with the microbubbles. Once again, these parameters may be determined automatically by the controller 108.

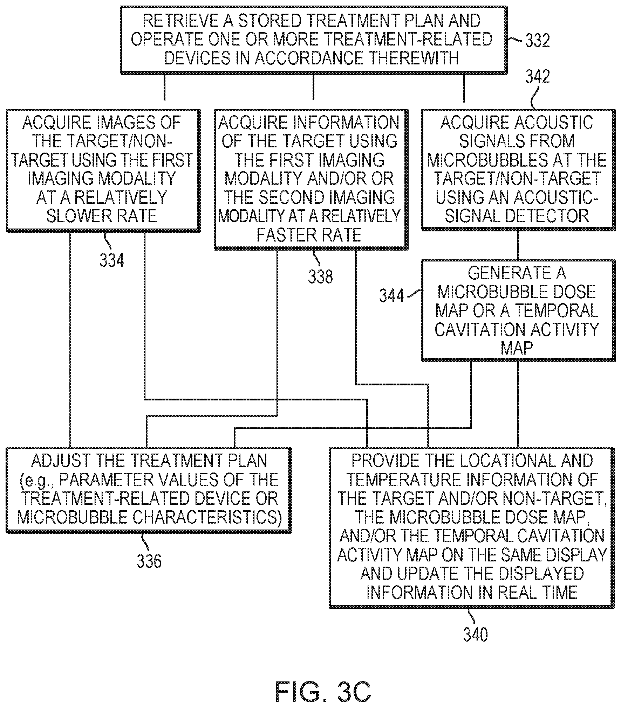

Referring to FIG. 3C, during the treatment procedure, the controller 108 may access the memory to retrieve the stored treatment plan and operate the transducer 102 and/or other treatment-related device (e.g., a microbubble administration device) in accordance therewith (in a step 332). For example, the transducer elements 104 may be activated in accordance with the parameter values specified in the treatment plan to transmit high-energy ultrasound pulses focused at the target for treatment (e.g., thermal ablation). In addition, the MRI system 200 may simultaneously acquire images of the target during treatment (in a step 334) and provide the image data as feedback to the controller 108; the controller 108 may then adjust the treatment plan (e.g., ultrasound parameter values and/or microbubble characteristics) based thereon so as to ensure treatment efficiency and safety (in a step 336). For example, if MR thermal imaging shows that the temperature at the target is below the desired ablation temperature, the controller may increase the amplitudes of the ultrasound waves. Conversely, if MR thermal imaging shows that the temperature at the non-target region exceeds its maximum tolerable temperature, the controller may reduce the ultrasound power. In addition, in cases where the MR imaging data indicates that the target has moved, the controller may determine updated ultrasound parameter values using the physical model so as to focus the ultrasound waves at the new target location.

To monitor treatment progress, it may be desirable to provide an image depicting both the target location and a temperature map of the target region on the same display. Although the MR imaging data may provide these two types of information, each type requires a detailed, high-resolution scan of the target that may require separate processing - e.g., creating a map of temperature differences may require reconstructing a real-space treatment image so as to identify the target location and subtracting the baseline phase image from the treatment image. Therefore, it is generally impractical to display simultaneously the locational and temperature information of the target utilizing the MR imaging data of the target only. To solve this problem, in various embodiments, operations of at least some of the transducer elements 104 are switched from a treatment mode to an imaging mode - i.e., instead of transmitting high-power ultrasound waves, the transducer elements 104 emit low-power waves (i.e., waves that do not cause any significant clinical effects on the target) and subsequently receive ultrasound reflections from the target (in a step 338). Again, each of the transducer elements may alternate between transmitting and receiving ultrasound waves. Alternatively, some of the transducer elements may transmit the low-power ultrasound waves while other transducer elements may receive the reflected waves at the same time. Based on the reflections, an image indicating the target location in real-time can be constructed in accordance with conventional techniques. The ultrasound images may be then registered with the MR thermal map so as to provide an image including both target location and target temperature in real time, thereby allowing the target movement and treatment process to be monitored at the same time during treatment.

In various embodiments, after the reflection signals are received by the transducer elements 104 and provided to the controller 108 for image processing, the elements 104 may be activated in the treatment mode again to transmit high-power ultrasound waves for treating the target. Because the ultrasound imaging procedure requires little time (e.g., 10 ms), the interruption for imaging produces no clinically significant effect on treatment of the target. In one implementation, the transducer elements 104 are switched from the treatment mode to the imaging mode periodically (e.g., every 100 ms) so as to provide real-time measurements of the target location during treatment. In addition - similar to the preparatory step 310 - during treatment, the target locations in two consecutive ultrasound images may be compared on a pixel-by-pixel basis to determine whether the target movement has occurred. If so (i.e., when the shift of target pixels between the two consecutive images exceeds the predetermined threshold), the phase map and/or the target location identified in the MR image acquired in step 334 is corrected to eliminate (or at least reduce) the artifacts resulting from the target movement. The corrected MR image may then provide more accuracy and better imaging resolution of the target for monitoring purposes. Again, the image correction may be facilitated utilizing imaging registration, a model or a look-up table as described above.

Additionally or alternatively, the target location may be monitored using one or more locational trackers (e.g., MR fiducials) (step 338). For example, after the MRI apparatus 200 acquires a high-resolution target image, and during processing of the data acquired to reconstructing a real-space image, the MRI apparatus 200 may take a fast, low-resolution scan of the MR fiducials. Again, because the MR fiducial data serves only to establish the target location, detailed image reconstruction thereof is unnecessary. The processing time to merely identify the spatial locations of the MR fiducials in the low-resolution, raw MR fiducial data and infer the target location therefrom may be relatively short. Accordingly, the target location can be obtained prior to or substantially simultaneously with generation of the MR thermal map using the high-resolution target image. This enables both locational and temperature information of the target to be provided on the same display and updated in real time (in a step 340). Again, the MR fiducials may be scanned periodically (e.g., every 5 target-image frames) to monitor the target location in real time during treatment.

In one embodiment, the target location is monitored using an additional object other than the MR fiducials. For example, a motion sensor (e.g., a respiration belt) 124 may be strapped around the patient to provide additional information about the patient's movement. This information may be complementary to the information provided by the fast-rate modality and/or MR fiducials for increasing accuracy of tracking the target location. Exemplary approaches to utilizing complementary information for enhancing tracking accuracy of the target location are provided in U.S. Patent Publication No. 2017/0358095. Additionally or alternatively, the fast-rate modality, slow-rate modality and/or a third imaging modality (e.g., a camera or a computed tomography device) may acquire images of the additional object (e.g., an anatomic landmark that can be identified in the third imaging modality) to provide image-related information about the patient's movement.

It should be noted that image acquisition of the target region using only one imaging modality (e.g., ultrasound imaging, MR imaging, or other suitable imaging modality) may be sufficient to track the target movement in real time. But because the ultrasound images may have less resolution than MR images and MR imaging may have a higher latency than ultrasound imaging, it may be preferable to combine them to provide high-resolution and short-latency images. Accordingly, various embodiments employ two imaging modalities having different rates (or one imaging modalities having two different rates) of acquiring information of the target/non-target. The information acquired at the slower rate may provide a more accurate location and/or thermal map of the target/non-target, while the information acquired at the faster rate may reduce information accuracy/resolution to allow faster motion tracking of the target/non-target in real time. By combining the information acquired at two rates, it is possible to create a treatment plan that, when executed, may efficiently and safely treat the target while avoiding damage to the non-target tissue. During treatment, information acquired using the two different rates may provide two distinct closed-loop feedback control signals - fast-loop and slow-loop data - to the controller 108. Based thereon, the controller 108 may continue or adjust the treatment plan (e.g., the ultrasound parameter values) to compensate for target motion in order to achieve the treatment goal. In addition, two imaging modalities may be required when a purpose other than tracking target movement (e.g., creation of a thermal map of the target and/or non-target regions and/or simultaneously display of the thermal map and target movement) is desired.

Referring again to FIG. 3C, the ultrasound treatment may involve microbubbles (for the purposes of autofocusing, BBB opening, and/or enhancing treatment effects). Thus, in various embodiments, the acoustic-signal detector 122 may be employed to measure acoustic signals from the microbubbles (in a step 342). Based on the measured microbubble signals (and optionally the MR imaging data (corrected or uncorrected), a microbubble dose map or a temporal cavitation activity map at the target and/or non-target regions may be generated (in a step 344). The controller 108 may then adjust the treatment plan (e.g., the ultrasound parameter values, the agent type, the size and/or the concentration of the microbubbles, the location of the microbubble injection site, and/or the administering profile (e.g., the administering dose or timing) associated with the microbubbles) based thereon. In addition, the generated microbubble dose map or a temporal cavitation activity map may be simultaneously provided with the MR thermal map and/or locational map of the target/non-target on the same display.

While the examples herein involve the delivery of ultrasound energy, it should be understood that these same systems and methods may be applied to other therapeutic modalities, such as radiation therapy, image-guided surgery, and others.

In general, functionality for creating a treatment plan and executing the treatment plan, including, for example, analyzing imaging data of the target and/or non-target regions acquired using one or more imaging modalities (e.g., ultrasound imaging, MR imaging or a camera), determining the target location, generating a baseline phase image based on the imaging data, tracking movement of the target tissue, non-target tissue and/or other non-target objects (e.g., locational trackers), creating a tissue model characterizing the material characteristics of the target/non-target regions based on the imaging data, computationally predicting movement of the target/non-target, correcting the target location and/or phase image based on the tracked/predicted target movement, determining the parameter values associated with the treatment-related device (e.g., the ultrasound transducer), activating the treatment-related device based on the determined values, acquiring acoustic signals from the microbubbles located at the target/non-target regions, generating a microbubble dose map or a temporal cavitation activity map based on the acquired microbubble signals, adjusting the treatment plan based on the tracked/predicted target movement and/or the acquired microbubble signals, and/or simultaneously providing locational and temperature information of the target/non-target, a microbubble dose map and/or a temporal cavitation activity map on the same display and updating the displayed information in real time, as described above, whether integrated within a controller of the imager 112 (e.g., MRI apparatus 200), and/or an ultrasound system 100, or provided by a separate external controller or other computational entity or entities, may be structured in one or more modules implemented in hardware, software, or a combination of both. The ultrasound controller 108 and/or MR controller 216 may include one or more modules implemented in hardware, software, or a combination of both. For embodiments in which the functions are provided as one or more software programs, the programs may be written in any of a number of high level languages such as PYTHON, FORTRAN, PASCAL, JAVA, C, C++, C#, BASIC, various scripting languages, and/or HTML. Additionally, the software can be implemented in an assembly language directed to the microprocessor resident on a target computer; for example, the software may be implemented in Intel 80x86 assembly language if it is configured to run on an IBM PC or PC clone. The software may be embodied on an article of manufacture including, but not limited to, a floppy disk, a jump drive, a hard disk, an optical disk, a magnetic tape, a PROM, an EPROM, EEPROM, field-programmable gate array, or CD-ROM. Embodiments using hardware circuitry may be implemented using, for example, one or more FPGA, CPLD or ASIC processors.

In addition, the term "controller" or "automatic control system" used herein broadly includes all necessary hardware components and/or software modules utilized to perform any functionality as described above; the controller may include multiple hardware components and/or software modules and the functionality can be spread among different components and/or modules.

## Claims

1. A system (100) for treating an anatomical target, the system comprising:
(a) a treatment apparatus;
(b) a first imaging modality (112) for acquiring one or more images of the anatomical target (114) during a time interval, the first imaging modality comprising an MRI apparatus;
(c) a second imaging modality (112) for acquiring a series of images of a first imageable object during the time interval, wherein the first imageable object has a relative location with respect to the anatomical target and the second imaging modality has a higher rate of image capture than the first imaging modality; and
(d) a controller (108) configured to (i) process the one or more images of the anatomical target so as to determine information associated with the target, (ii) process the series of images of the first imageable object, (iii) track movement of the anatomical target based on the series of images and the relative location of the first imageable object with respect to the anatomical target, and (iv) based at least in part on the tracked movement and the information associated with the anatomical target during the time interval, generate a treatment plan associated with the treatment apparatus for treating the anatomical target,
wherein the controller is further configured to update the information associated with the anatomical target based on the tracked movement thereof,
**characterised in that** the one or more images of the anatomical target comprise one or more baseline phase images, and the controller is further configured to subtract a phase shift associated with the tracked movement from the one or more baseline phase images so as to generate a thermal map of the anatomical target.

2. The system of claim 1, wherein the controller (108) is further configured to activate the treatment apparatus based at least in part on the treatment plan.

3. The system of claim 1, wherein the first (112) or second (112) imaging modality is further configured acquire a second series of images of a second imageable object, and the controller (108) is further configured to track movement of the anatomical target (114) based at least in part on the second series of images of the second imageable object.

4. The system of claim 1, further comprising a third imaging modality (112) for acquiring a second series of images of a second imageable object, the controller (108) being further configured to track movement of the anatomical target (114) based at least in part on the second series of images of the second imageable object.

5. The system of claim 1, wherein the controller (108) is further configured to track movement of the anatomical target (114) based at least in part on a predicted movement of the anatomical target.

6. The system of claim 1, wherein the controller (108) is further configured to track movement of the anatomical target (114) based at least in part on a predicted movement of a second imageable object.

7. The system of claim 1, wherein the first imageable object comprises the anatomical target (114).

8. The system of claim 1, wherein the first imageable object is different from the anatomical target (114), and the controller (108) is further configured to determine the location of the first imageable object in each of the series of images acquired in step (c), and infer a location of the anatomical target (114) based on the determined location of the first imageable object.

9. The system of claim 8, wherein the first imageable object is an anatomical feature, or an MR tracker.

10. The system of claim 1, wherein the information associated with the anatomical target (114) comprises at least one of a location, a thermal map, a microbubble dose map, or a temporal cavitation activity map thereof.

11. The system of claim 1, wherein the first imaging modality (112) further acquires a reference library comprising a series of reference images of the first imageable object,
optionally wherein the controller (108) is further configured to (i) compare one of the images of the first imageable object acquired using the second imaging modality (112) against the reference images in the reference library to identify a best-matching reference image and (ii) determine the phase shift based at least in part on the identified best-matching reference image.

12. The system of claim 1, wherein the second (112) imaging modality comprises an MRI apparatus (200), wherein the MRI apparatus is configured to acquire the one or more images of the anatomical target (114) at a relatively faster scanning rate and to acquire the series of images of the first imageable object at a relatively slower scanning rate.

13. The system of claim 1, wherein the second imaging modality comprises a camera.

14. The system of claim 1, wherein the second imaging modality comprises an ultrasound apparatus.

15. The system of claim 14, wherein the treatment apparatus comprises
a) the ultrasound apparatus;
b) a second ultrasound apparatus different from the second imaging modality; or
c) a plurality of transducer elements (104), and the treatment plan specifies at least one of an amplitude, a frequency, a phase, a direction or an activation time associated with at least one of the transducer elements.

## Patentansprüche

1. System (100) zum Behandeln eines anatomischen Ziels, wobei das System umfasst:
a) eine Behandlungsvorrichtung;
b) eine erste Bildgebungsmodalität (112) zum Erfassen eines oder mehrerer Bilder des anatomischen Ziels (114) während eines Zeitintervalls, wobei die erste Bildgebungsmodalität eine MRT-Vorrichtung umfasst;
c) eine zweite Bildgebungsmodalität (112) zum Erfassen einer Reihe von Bildern eines ersten abbildbaren Objekts während des Zeitintervalls, wobei das erste abbildbare Objekt eine relative Position in Bezug auf das anatomische Ziel aufweist und die zweite Bildgebungsmodalität eine höhere Bilderfassungsrate aufweist als die erste Bildgebungsmodalität; und
d) eine Steuerung (108), die konfiguriert ist, um (i) das eine oder die mehreren Bilder des anatomischen Ziels zu verarbeiten, um dem Ziel zugeordnete Informationen zu bestimmen, (ii) die Reihe von Bildern des ersten abbildbaren Objekts zu verarbeiten, (iii) die Bewegung des anatomischen Ziels basierend auf der Reihe von Bildern und dem relativen Ort des ersten abbildbaren Objekts in Bezug auf das anatomische Ziel zu verfolgen und (iv) mindestens teilweise basierend auf der verfolgten Bewegung und den dem anatomischen Ziel zugeordneten Informationen während des Zeitintervalls einen der Behandlungsvorrichtung zugeordneten Behandlungsplan zur Behandlung des anatomischen Ziels zu erzeugen,
wobei die Steuerung ferner konfiguriert ist, um die dem anatomischen Ziel zugeordneten Informationen basierend auf der verfolgten Bewegung desselben zu aktualisieren,
**dadurch gekennzeichnet, dass** das eine oder die mehreren Bilder des anatomischen Ziels ein oder mehrere Grundlinien-Phasenbilder umfassen und die Steuerung ferner konfiguriert ist, um eine der verfolgten Bewegung zugeordnete Phasenverschiebung von dem einen oder den mehreren Grundlinien-Phasenbildern zu subtrahieren, um eine Wärmekarte des anatomischen Ziels zu erzeugen.

2. System nach Anspruch 1, wobei die Steuerung (108) ferner konfiguriert ist, um die Behandlungsvorrichtung mindestens teilweise basierend auf dem Behandlungsplan zu aktivieren.

3. System nach Anspruch 1, wobei die erste (112) oder zweite (112) Bildgebungsmodalität ferner konfiguriert ist, um eine zweite Reihe von Bildern eines zweiten abbildbaren Objekts zu erfassen, und die Steuerung (108) ferner konfiguriert ist, um die Bewegung des anatomischen Ziels (114) mindestens teilweise basierend auf der zweiten Reihe von Bildern des zweiten abbildbaren Objekts zu verfolgen.

4. System nach Anspruch 1, ferner umfassend eine dritte Bildgebungsmodalität (112) zum Erfassen einer zweiten Reihe von Bildern eines zweiten abbildbaren Objekts, wobei die Steuerung (108) ferner konfiguriert ist, um die Bewegung des anatomischen Ziels (114) mindestens teilweise basierend auf der zweiten Reihe von Bildern des zweiten abbildbaren Objekts zu verfolgen.

5. System nach Anspruch 1, wobei die Steuerung (108) ferner konfiguriert ist, um die Bewegung des anatomischen Ziels (114) mindestens teilweise basierend auf einer vorhergesagten Bewegung des anatomischen Ziels zu verfolgen.

6. System nach Anspruch 1, wobei die Steuerung (108) ferner konfiguriert ist, um die Bewegung des anatomischen Ziels (114) mindestens teilweise basierend auf einer vorhergesagten Bewegung eines zweiten abbildbaren Objekts zu verfolgen.

7. System nach Anspruch 1, wobei das erste abbildbare Objekt das anatomische Ziel (114) umfasst.

8. System nach Anspruch 1, wobei sich das erste abbildbare Objekt von dem anatomischen Ziel (114) unterscheidet und die Steuerung (108) ferner konfiguriert ist, um den Ort des ersten abbildbaren Objekts in jeder der Reihe von Bildern, die in Schritt (c) erfasst wurden, zu bestimmen und einen Ort des anatomischen Ziels (114) basierend auf dem bestimmten Ort des ersten abbildbaren Objekts abzuleiten.

9. System nach Anspruch 8, wobei das erste abbildbare Objekt ein anatomisches Merkmal oder ein MR-Tracker ist.

10. System nach Anspruch 1, wobei die dem anatomischen Ziel (114) zugeordneten Informationen mindestens eines von einem Ort, einer Wärmekarte, einer Karte der Mikrobläschendosis oder einer Karte der zeitlichen Kavitationsaktivität davon umfassen.

11. System nach Anspruch 1, wobei die erste Bildgebungsmodalität (112) ferner eine Referenzbibliothek erfasst, die eine Reihe von Referenzbildern des ersten abbildbaren Objekts umfasst, wobei gegebenenfalls die Steuerung (108) ferner konfiguriert ist, um (i) eines der Bilder des ersten abbildbaren Objekts, das unter Verwendung der zweiten Bildgebungsmodalität (112) erfasst wurde, mit den Referenzbildern in der Referenzbibliothek zu vergleichen, um ein am besten übereinstimmendes Referenzbild zu identifizieren, und (ii) die Phasenverschiebung mindestens teilweise basierend auf dem identifizierten, am besten übereinstimmenden Referenzbild zu bestimmen.

12. System nach Anspruch 1, wobei die zweite Bildgebungsmodalität (112) eine MRT-Vorrichtung (200) umfasst, wobei die MRT-Vorrichtung konfiguriert ist, um das eine oder die mehreren Bilder des anatomischen Ziels (114) mit einer relativ schnelleren Abtastrate zu erfassen und die Reihe von Bildern des ersten abbildbaren Objekts mit einer relativ langsameren Abtastrate zu erfassen.

13. System nach Anspruch 1, wobei die zweite Bildgebungsmodalität eine Kamera umfasst.

14. System nach Anspruch 1, wobei die zweite Bildgebungsmodalität eine Ultraschallvorrichtung umfasst.

15. System nach Anspruch 14, wobei die Behandlungsvorrichtung umfasst
a) die Ultraschallvorrichtung;
b) eine zweite Ultraschallvorrichtung, die sich von der zweiten Bildgebungsmodalität unterscheidet; oder
c) eine Vielzahl von Wandlerelementen (104), und der Behandlungsplan mindestens eine Amplitude, eine Frequenz, eine Phase, eine Richtung oder eine Aktivierungszeit, die mindestens einem der Wandlerelemente zugeordnet ist, spezifiziert.

## Revendications

1. Système (100) permettant de traiter une cible anatomique, le système comprenant :
a) un appareil de traitement ;
b) une première modalité d'imagerie (112) permettant d'acquérir au moins une image de la cible (114) anatomique pendant un intervalle temporel, la première modalité d'imagerie comprenant un appareil d'IRM ;
c) une deuxième modalité d'imagerie (112) permettant d'acquérir une série d'images d'un premier objet pouvant être imagé pendant l'intervalle temporel, dans lequel le premier objet pouvant être imagé présente un emplacement relatif par rapport à la cible anatomique et la deuxième modalité d'imagerie présente une fréquence de capture d'image supérieure à la fréquence de capture d'image de la première modalité d'imagerie ; et
d) un dispositif de commande (108) conçu (i) pour traiter l'au moins une image de la cible anatomique de manière à déterminer des informations associées à la cible, (ii) pour traiter la série d'images du premier objet pouvant être imagé, (iii) pour suivre le mouvement de la cible anatomique en fonction de la série d'images et de l'emplacement relatif du premier objet pouvant être imagé par rapport à la cible anatomique, et (iv) pour générer, en fonction, au moins partiellement, du mouvement suivi et des informations associées à la cible anatomique pendant l'intervalle temporel, un plan de traitement associé à l'appareil de traitement permettant de traiter la cible anatomique,
dans lequel le dispositif de commande est en outre conçu pour mettre à jour les informations associées à la cible anatomique en fonction du mouvement suivi de la cible,
**caractérisé en ce que** l'au moins une image de la cible anatomique comprend au moins une image de phase de référence, et le dispositif de commande est en outre conçu pour soustraire un déphasage associé au mouvement suivi de l'au moins une image de phase de référence de manière à générer une carte thermique de la cible anatomique.

2. Système selon la revendication 1, dans lequel le dispositif de commande (108) est en outre conçu pour activer l'appareil de traitement en fonction, au moins partiellement, du plan de traitement.

3. Système selon la revendication 1, dans lequel la première (112) ou la seconde (112) modalité d'imagerie est en outre conçue pour acquérir une seconde série d'images d'un second objet pouvant être imagé, et le dispositif de commande (108) est en outre conçu pour suivre le mouvement de la cible (114) anatomique en fonction, au moins partiellement, de la seconde série d'images du second objet pouvant être imagé.

4. Système selon la revendication 1, comprenant en outre une troisième modalité d'imagerie (112) permettant d'acquérir une seconde série d'images d'un second objet pouvant être imagé, le dispositif de commande (108) étant en outre conçu pour suivre le mouvement de la cible (114) anatomique en fonction, au moins partiellement, de la seconde série d'images du second objet pouvant être imagé.

5. Système selon la revendication 1, dans lequel le dispositif de commande (108) est en outre conçu pour suivre le mouvement de la cible (114) anatomique en fonction, au moins partiellement, d'un mouvement prédit de la cible anatomique.

6. Système selon la revendication 1, dans lequel le dispositif de commande (108) est en outre conçu pour suivre le mouvement de la cible (114) anatomique en fonction, au moins partiellement, d'un mouvement prédit d'un second objet pouvant être imagé.

7. Système selon la revendication 1, dans lequel le premier objet pouvant être imagé comprend la cible (114) anatomique.

8. Système selon la revendication 1, dans lequel le premier objet pouvant être imagé est différent de la cible (114) anatomique, et le dispositif de commande (108) est en outre conçu pour déterminer l'emplacement du premier objet pouvant être imagé dans chacune des séries d'images acquises à l'étape (c), et pour déduire un emplacement de la cible (114) anatomique en fonction de l'emplacement déterminé du premier objet pouvant être image.

9. Système selon la revendication 8, dans lequel le premier objet pouvant être imagé est une caractéristique anatomique ou un appareil de suivi MR.

10. Système selon la revendication 1, dans lequel les informations associées à la cible (114) anatomique comprennent un emplacement et/ou une carte thermique et/ou une carte de dose de microbulles et/ou une carte d'activité de cavitation temporelle de la cible.

11. Système selon la revendication 1, dans lequel la première modalité d'imagerie (112) acquiert en outre une bibliothèque de référence comprenant une série d'images de référence du premier objet pouvant être imagé, éventuellement dans lequel le dispositif de commande (108) est en outre conçu (i) pour comparer l'une des images du premier objet pouvant être imagé acquis à l'aide de la deuxième modalité d'imagerie (112) avec les images de référence dans la bibliothèque de référence pour identifier une image de référence la mieux adaptée et (ii) pour déterminer le déphasage en fonction, au moins partiellement, de l'image de référence la mieux adaptée identifiée.

12. Système selon la revendication 1, dans lequel la deuxième modalité d'imagerie (112) comprend un appareil d'IRM (200), dans lequel l'appareil d'IRM est conçu pour acquérir l'au moins une image de la cible (114) anatomique à une vitesse de balayage relativement supérieure et pour acquérir la série d'images du premier objet pouvant être imagé à une vitesse de balayage relativement inférieure.

13. Système selon la revendication 1, dans lequel la deuxième modalité d'imagerie comprend une caméra.

14. Système selon la revendication 1, dans lequel la deuxième modalité d'imagerie comprend un appareil à ultrasons.

15. Système selon la revendication 14, dans lequel l'appareil de traitement comprend
a) l'appareil à ultrasons ;
b) un second appareil à ultrasons différent de la deuxième modalité d'imagerie ; ou
c) une pluralité d'éléments transducteurs (104), et le plan de traitement spécifie une amplitude et/ou une fréquence et/ou une phase et/ou une direction et/ou un temps d'activation associés à l'au moins un des éléments transducteurs.
